# EUROPEAN PATENT SPECIFICATION

(11) **EP 1 309 266 B1**
(45) Date of publication and mention of the grant of the patent: **17.07.2013**
(21) Application number: 00952704.5
(22) Date of filing: 10.08.2000
(51) Int. Cl.: A61B 1/00, B29C 51/36, A61M 25/00

(54) **APPARATUS AND METHOD FOR FORMING COMPLEX-SHAPED COMPONENTS IN A HEATED POLYMERIC FILM**
VERFAHREN UND VORRICHTUNG ZUR FORMUNG VON KOMPONENTEN MIT KOMPLIZIERTER GESTALT IN EINER ERHITZTEN KUNSTSTOFFFOLIE
PROCEDE DE PRODUCTION DE COMPOSANTS DE FORME COMPLEXE DANS UN FILM POLYMERE CHAUFFE ET APPAREIL CORRESPONDANT

(43) Date of publication of application: 14.05.2003
(73) Proprietor: Vision Sciences, Inc., Natick, MA 01760 (US)
(72) Inventor: HARHEN, E., Paul, Duxbury, MA 02332 (US); GODDARD, James, M., Pepperell, MA 01463 (US)
(74) Representative: Klunker . Schmitt-Nilson . Hirsch
(86) International application number: PCT/US2000/021819
(87) International publication number: WO 2002/013681

(56) References cited:
- WO-A-00/42900
- US-A- 4 059 380
- US-A- 4 123 215
- US-A- 4 397 808
- US-A- 5 443 781
- US-A- 5 902 246
- US-A1- 4 459 255
- US-A1- 5 125 143
- US-A1- 5 443 781

## Description

### TECHNICAL FIELD

The present invention is directed toward a method of making medical components, and more particularly toward a method of making contoured, thin-walled, complex-shaped medical components usable with intrabody medical devices.

### BACKGROUND OF THE INVENTION

The use of intrabody medical equipment, such as endoscopes, catheters, and the like, for diagnostic and therapeutic indications is rapidly expanding. To improve performance, the equipment has been optimized to best accomplish the selected purpose. As an example, endoscopes have been optimized and refined so as to provide upper endoscopes for the examination of the esophagus, stomach, and duodenum, colonoscopes for examining the colon, angioscopes for examining blood vessels, bronchoscopes for examining the bronchii, laparoscopes for examining the peritoneal cavity, arthroscopes for examining joints spaces, nasopharygoscopes for examining nasal passages and pharynx, and intubation scopes for examination of a person's airway.

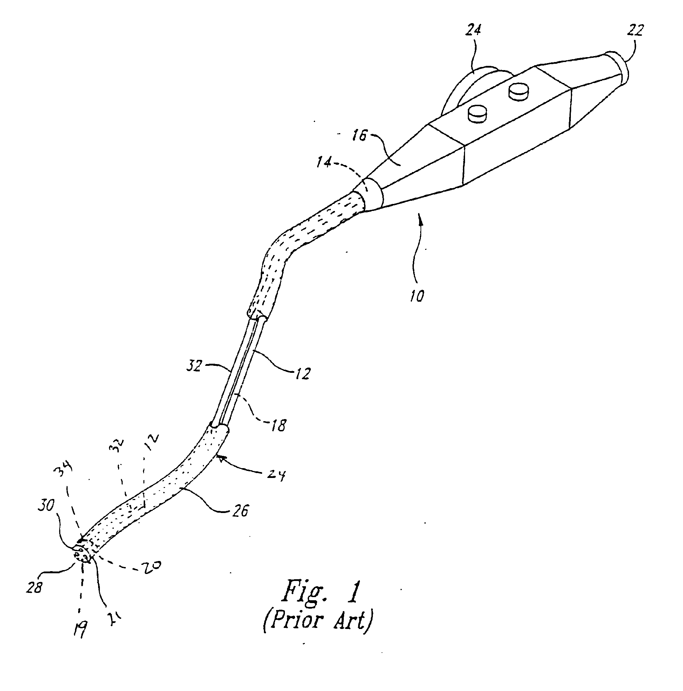

Optimization of the intrabody medical devices for such therapeutic and diagnostic procedures has resulted in sterile, inexpensive disposable equipment that is used alone or with non-disposable equipment. In the field of endoscopes, a conventional endoscope 10, shown in Figure 1, has an insertion tube 12 that is connected at its proximal end 14 to a handle or control body 16. The insertion tube 12 is adapted to be inserted into a patient's body cavity to perform a selected therapeutic or diagnostic procedure. The insertion tube 12 contains an imaging system 18 having optical fibers or the like extending along the length of the insertion tube and terminating at a viewing window 19 in the insertion tube's distal end 20. The imaging system 18 conveys an image from the viewing window 19 to an eyepiece 22 on the control body 16 or to a monitor (not shown), so the user can see into a selected body cavity during an endoscopic procedure. The endoscope 10 is described in greater detail in U.S. Patent No. Re 34,110 (US 4,869,238) and U.S. Patent No. 4,646,722.

Disposable endoscopic sheath assemblies are used to cover the insertion tube 12 and protect it from being contaminated. Accordingly, the sheath assembles alleviate the problem and cost of cleaning and sterilizing the insertion tube 12 between endoscopic procedures. As seen in Figure 1, a conventional sheath assembly 24, shown partially cut away for illustrative purposes, includes a flexible, elastic sheath 26 that tightly surrounds the endoscope's insertion tube 12. The sheath 26 may also contain one or more working channels 32 that extend along the insertion tube 12 and that are adapted to receive conventional endoscopic accessories therethrough without contaminating the endoscope itself during the endoscopic procedure. The sheath 26 has a distal end portion 21 that includes an endcap 34 having a transparent window 28 positioned to cover the viewing window 19 at the insertion tube's distal end 20 when the sheath assembly is installed. The endcap 34 formed of a relatively rigid material and is sealably secured to the sheath's distal end portion 21.

Endoscopic sheath assemblies used with insertion tubes that have a complex cross-sectional shape, such as C-shaped, often must have an endcap with a corresponding complex cross-sectional shape to snugly fit over the insertion tube's distal end 20. The complex-shaped endcap can be a costly and laborious component to manufacture for the sheath assembly. Other thin-walled components that would be required to have a complex shape if used with endoscopic sheaths or other medical devices having complex shapes may include precision detent mechanisms and short-pitched threads that are manufactured with the necessary accuracy and tolerances required for intricate medical devices.

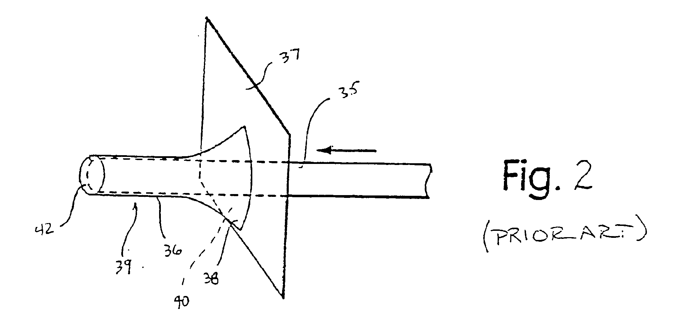

U.S. Patent No. 5,443,781 to Saab teaches a method of forming a disposable sheath with an optically transparent window that is integral with the sheath. The transparent window is formed so as to maintain a thin-walled, relatively inelastic, yet flexible sheath for an optical medical instrument. Saab teaches forming the sheath by heating a sheet or film of optically transparent polymeric material until the material's viscosity is substantially reduced and the film is malleable. As shown in Figure 2, a mandrel 35 having a relatively simply cylindrical shape is thrust into the heated film 37 causing the film to stretch and to generally conform to the mandrel's shape. As a result, the heated film 37 is formed into a closed-end sheath 39 having sidewalls 36, a flange or collar 38 at its open proximal end 40, and a closed distal end 42. The mandrel 35 is selected based upon its simple geometrical shape to define the shape of the sheath 39. Accordingly, sheaths having different shapes and sizes are formed using different mandrels with the corresponding shapes and sizes.

The sheath and technique of making the sheath as discussed in Saab, however, results in the film being heated, stretched, and then cooled to remain the shape that generally corresponds to the mandrel's simple. The resulting sheath is limited to having a relatively simple geometrical shape. The method of Saab does not sufficiently allow for making complex-shaped components having close tolerances.

WO 00/42900 A shows a thin-walled elastic sheath that can be stretched axially over an elongated imaging device to closely conform to the device and isolate the device from an external environment, and a method of forming such a sheath, are shown and described. The method includes the steps of heating at least a portion of a sheet of an elastomeric material to an elevated temperature to form a malleable heated portion of the sheet, pressing an elongated forming tool against the sheet at a location central with respect to the heated portion of the sheet, stretching the heated portion of the elastomeric material with the forming tool until an elastic conforming portion of the sheet is conformed to at least a portion of the length of the forming tool, and removing the forming tool from the conforming portion of the sheet to leave the thin-walled elastic sheath having a wall thickness approximately equal to or less than 0.006 inches.

In the design of intrabody medical devices and accessories, including optical and non-optical devices, the need for components having a complex geometry has become more and more apparent. As an example, there is a need for highly-detailed components, such as endcaps, used with more complicated endoscopes. There is further a need for precisely manufactured catheter components or the like with accurately positioned detents or close-pitched threads. Other medical devices and accessories would be benefited by inexpensive thin-walled components with close tolerance demands.

### SUMMARY OF THE INVENTION

The present invention provides a method capable of forming complex-shaped medical components in a heated polymeric film to provide thin-walled, durable, low-cost components that may be manufactured to close tolerances, which has been problematic in the prior art. In an exemplary embodiment of the present invention, the method of forming a thin-walled polymeric component includes the steps of heating a portion of the polymeric film to a malleable temperature, and using a forming tool to form a protrusion in the film's heated portion while the polymeric film is malleable. The formed protrusion has an open proximal end, a distal end spaced apart from the proximal end, and sidewalls extending between the proximal and distal ends. The sidewalls and distal end define a first shape of the protrusion and also define an interior of the protrusion. The forming tool has a shaping portion that may have a selected complex shape that is different than the protrusion's first shape. The method further includes the step of exerting a force radially inwardly on the protrusion and moving the protrusion into firm engagement with the forming tool's shaping portion so the protrusion moves from the first shape to a second shape that closely conforms to the selected shape of the forming tool's shaping portion.

In the exemplary embodiment, the radially inward force exerted on the protrusion is achieved by creating a differential pressure so the pressure within the interior of the protrusion is less than the pressure outside the protrusion, thereby causing the protrusion's sidewalls to move inwardly to the second shape. In one embodiment, the step of exerting a differential pressure on the protrusion is achieved by drawing a partial vacuum inside the protrusion. The partial vacuum may be drawn through the forming tool and through at least one vacuum port in the forming tool.

The present invention is also directed toward a polymeric medical component that may have a selected complex shape that is made by the above-described process.

The present invention is also directed toward a vacuum-forming tool capable of forming the medical component from a malleable polymeric protrusion. The vacuum-forming tool includes a shaft having an air passageway and a forming portion, and the forming portion has a shape corresponding to the selected shape of the medical component. The forming portion has an outer surface and a plurality of aperrures extending from the outer surface to the air passageway. The shaft is connectable to a vacuum source so the air passageway communicates with the vacuum source to allow the vacuum source to draw air through the apertures into the air passageway, thereby forming a partial vacuum within the malleable polymeric protrusion during the formation of the medical component.

### BRIEF DESCRIPTION OF THE DRAWINGS

Figure 1 is an isometric view of a prior art endoscope and endoscopic sheath assembly.
Figure 2 is an isometric view of a thermal plastic film of the prior art stretched by a mandrel.
Figure 3 is an isometric view of a distal portion of an endoscopic sheath having a thin-walled, polymeric endcap formed in accordance with one embodiment of the present invention.
Figure 4 is an isometric view of a heated thin-walled polymeric film with a funnel-shaped projection formed therein in accordance with one embodiment of the present invention and shown in an extended position, and a complex-shaped forming tool is shown positioned in the protrusion and connected to a vacuum source.
Figure 5 is an isometric view of the heated polymeric film of Figure 4 with the projection shown in a contracted position in solid lines on the forming tool, and the projection is shown in the extended position in phantom lines.
Figure 6 is an isometric view of the protrusion of Figure 5 shown with a distal portion of the protrusion being separated from a proximal to provide the selected thin-walled polymeric endcap with complex shape.
Figure 7 is an isometric view of an alternate embodiment of the present invention with the thin-walled component having close-pitched threads formed therein by a complex-shaped threaded forming tool shown in phantom lines.
Figure 8 is an isometric view of an alternate embodiment of the present invention with the thin-walled component having detents formed therein by a forming tool shown in phantom lines.

### DETAILED DESCRIPTION OF THE INVENTION

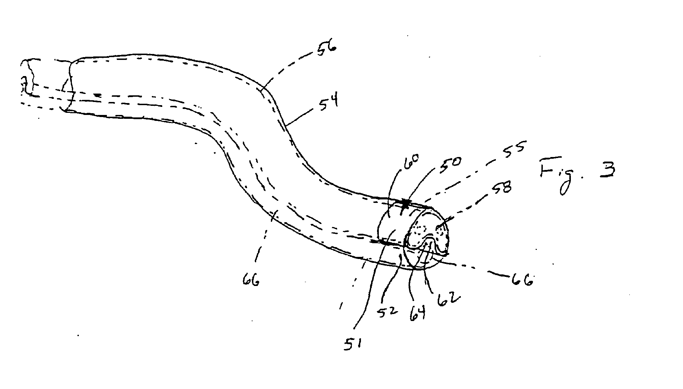

A thin-walled polymeric medical component 50 having a complex shape made in accordance with one embodiment of the present invention is described herein with reference to the attached drawings. An exemplary embodiment of a thin-walled medical component 50, shown in Figure 3, is attached to a distal end 52 of a sheath 54. The sheath 54 is shaped and sized to receive and isolate, as an example, an endoscope insertion tube 56 shown in phantom lines. The illustrated medical component 50 is a sheath endcap 51 that is sealably attached to the sheath's distal end 52 so as to provide a viewing window therein that allows clear imaging through the endoscope's imaging system 58, also shown in phantom lines.

The distal end portion 55 of the illustrated insertion tube 56 has a generally C-shaped cross-section and the endcap 51 is formed with a corresponding complex geometrical shape that closely conforms to the insertion tube's distal end portion. More specifically, the C-shaped cross-section of the illustrated endcap 51 is defined by a curved outer axial wall 60 that has a selected radius and a curved inner axial wall 62 that is integrally connected to the outer axial wall 60. The inner axial wall 62 has a radius which is smaller than the radius of the outer axial wall 60. The inner axial wall 62 defines a receiving area 64 that is shaped and sized to receive one or more selected working channels 66, shown in phantom lines, extending through the sheath 54. As an example, the illustrated endoscopic sheath 54 includes a tubular working channel 66 that extends through the sheath and that is sealably connected to the sheath's distal end 52. The tubular working channel 56 snugly fits in the endcap's receiving area 64, while the distal end of the endoscope's insertion tube 56 is snugly received in the endcap 51 between the inner and outer walls. Accordingly, the thin-walled endcap 51 is provided with a complex geometrical shape that receives and retains the insertion tube's distal end therein and retains the working channel 66 adjacent to the distal end while minimizing the cross-sectional area of the sheath and insertion tube combination which is to be inserted into a patient.

While the medical component 50, such as the end cap 51, and the method of making the component is discussed herein with reference to an endoscopic sheath, the method of the present invention is equally applicable to medical components used in procedures for medical devices other than endoscopic procedures. As an example, the medical component 50 in alternate embodiments are complex-shaped components used with catheters, optical medical equipment, and non-optical imaging medical equipment.

The thin-walled polymeric medical component 50 is made by forming a thin polymeric film into the desired shape. The preferred polymeric film used with the present invention is a thermoplastic material including PVC, acrylic, polycarbonate, polyethylene terephthalate, or other thermoplastic polyester material. The polymeric 5 material used by the present invention allows for thin-walled medical components that are substantially inelastic, flexible, and have-high strength, and that can be easily, inexpensively, and quickly manufactured. The polymeric film is selected so the medical component 50 has a wall thickness in the range of approximately 0.0127 mm to 0.381 mm (0.0005 to 0.015 inches), inclusive, and preferably in the range of approximately 0.0203 mm to 0.127 mm (0.0008 to 0.005 inches), inclusive.

In the exemplary embodiment illustrated in Figures 3, 4 and 5, the medical component 50 is formed from a material that is optically transparent to visible light. The polymeric film however, may be selected to provide a desired degree of transparency to electromagnetic radiation in the visible spectrum, or in the non-visible spectrum, *e.g*., radar, laser, or microwave. The polymeric flm may also be selected, when needed, to provide a desired degree of transparency to sound waves, such as for use with ultrasound devices.

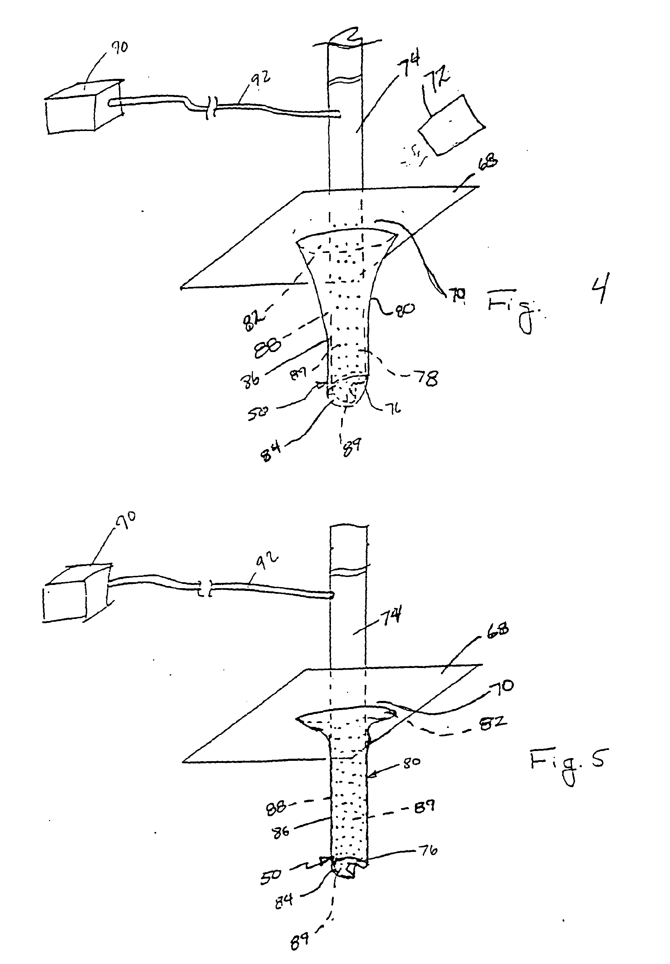

As best seen in Figure 4, the polymeric film 68 is rerained in a selected position so as to substantially define a plane. An interior portion of the film 68 is heated by a conventional heating device 72 to a selected malleable temperature to form a malleable, heated portion 70 of the film. The heated portion 70 is then stretched and formed into a protrusion 80, as discussed in greater detail below, by a forming tool 74. The forming tool 74 has a contoured shaping portion 78 with a complex geometrical shape that corresponds to the shape of the endcap or other medical component to be formed. In the illustrated embodiment, the forming tool's shaping portion 78 has a C-shaped cross-section with curved inner and outer surfaces that correspond to the endcap's C-shaped cross-section discussed above.

After the film's heated portion 70 is malleable, the forming tool's shaping portion 78 is pressed into the heated portion generally perpendicular to the plane defined by the film 68, and the forming tool stretches the heated portion away from the plane to form a thin-walled, generally funnel-shaped protrusion 80.

The protrusion 80 has an open proximal end 82, a closed distal end 84 spaced away from the open proximal end and sidewalls 86 extending between the proximal and distal ends. The closed distal end 84 and the sidewalls 86 define an interior 88 of the protrusion 80 that contains the forming tool's shaping portion 78 as the protrusion is formed.

As the protrusion 80 is formed, the protrusion has a generally tubular shape that only roughly conforms to the outer shape of the forming tool's shaping portion 78, but the protrusion's sidewalls 86 do not yet closely conform to the complex shape of the forming tool's shaping portion. As an example, the protrusion's sidewalls 86 conform to the shaping portion's outer curved surface but do not conform to the shaping portion's inner curved surface, such that the protrusion has a generally D-shaped cross-section, rather than the C-shaped cross-section of the shaping portion.

While still malleable, the protrusion's sidewalls 86 and the closed distal end 84 are then brought into firm and close engagement with the forming tool's shaping portion 78, as shown in Figure 5, thereby closely conforming to the shaping portion's complex geometrical shape. In the exemplary embodiment, the protrusion 80 is brought into firm engagement with the shaping portion 78 by drawing a partial vacuum within the projection's interior 88. The partial vacuum causes a differential pressure in the protrusion that is exerted on the sidewalls 86, so the pressure within the protrusion is less than the pressure outside of the protrusion. Accordingly, the partial vacuum moves the protrusion's sidewalls 86 against the forming tool's shaping portion 78 to achieve the selected complex geometrical shape for the medical component 50. After the protrusion 80 has moved to the contracted position and has cooled from the malleable temperature, the forming tool 74 is removed from the protrusion's interior 88 and the protrusion remains in the contracted position.

As best seen in Figures 4 and 5, the forming tool 74 is a partially hollow member with at least one and preferably a plurality of vacuum ports 89 formed in the shaping portion 78. The forming tool 74 is connected to a conventional vacuum source 90 by a vacuum line 92. The vacuum source is adapted to draw air into the forming tool's shaping portion 78 through the vacuum ports 89, thereby creating the partial vacuum within the interior.

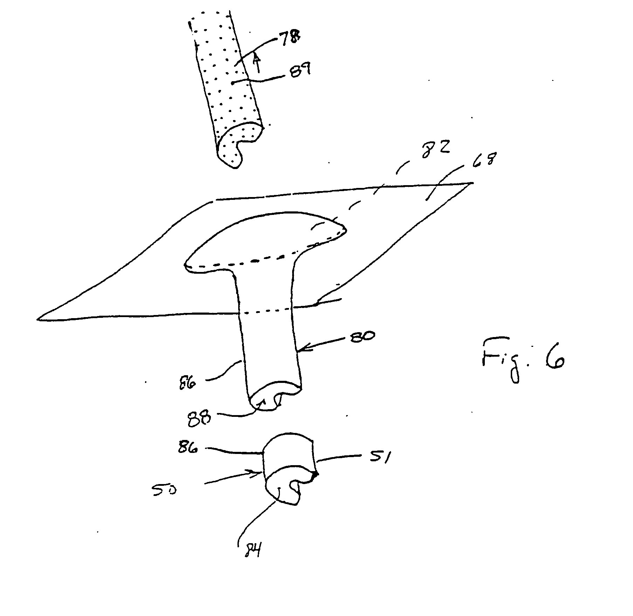

As best seen in Figure 6, after the forming tool 74 is removed, a distal portion of the protrusion 80, which has the complex-shape formed therein, is cut or otherwise separated from the remaining portion of the protrusion, and that distal portion is the thin-walled polymeric medical component 50. The component 50, such as the illustrated C-shaped endcap 51, is then connected to the selected medical device, such as the distal end 52 of the endoscopic sheath 54, as illustrated in Figure 3.

In an alternate embodiment (not shown), the protrusion 80 is moved to the contracted position by exerting substantially uniform, exterior pressure on the protrusion in a radially inward direction. As an example, the distal portion of the protrusion 80 and the forming tool's shaping portion 78 are positioned, as a unit, within a pneumatic pressure-generating device or other suitable device that exerts the exterior pressure, and the pressure generating device moves the protrusion's sidewalls 86 from the expanded position to the contracted position and into close engagement with the forming tool's shaping portion 78. Accordingly, the radially inward directed forces on the protrusion 80 may be achieved by generating a vacuum in the protrusion's interior or by creating a pressure external to the protrusion. The preferred method, however, is to use the partial vacuum within the protrusion's interior 88 because the partial vacuum does not disturb the temperature profile of the heated polymeric film.

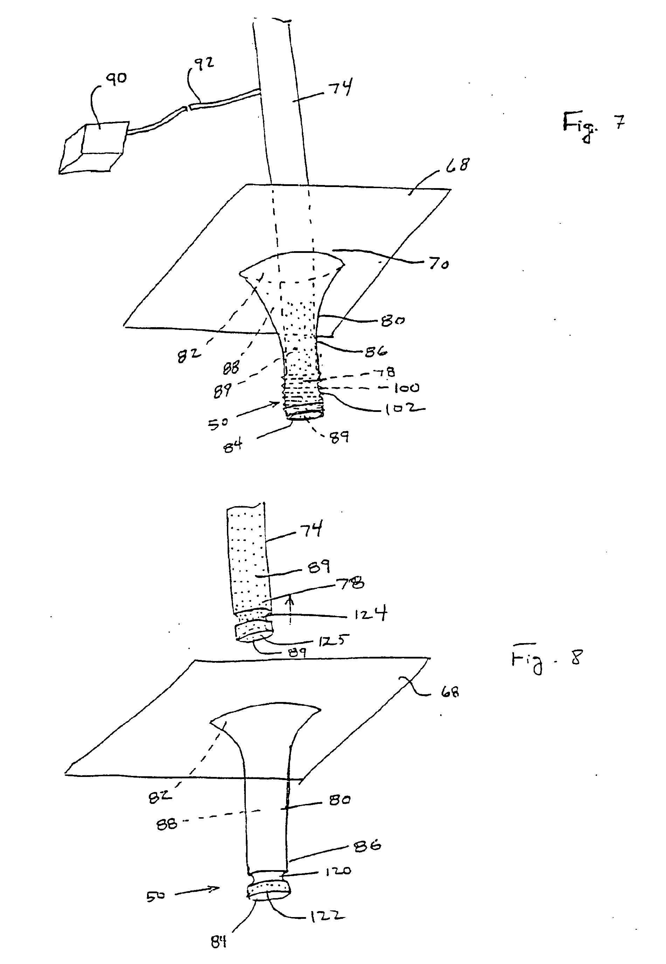

In addition to forming medical components having complex contoured shapes, such as the C-shape cross-sectional shape, the disclosed embodiment of the present invention also allows easy and inexpensive formation of a thin-walled, polymeric medical component 50 having close-pitched, accurately-located threads formed therein. As best seen in Figure 7, the forming tool 74 has a plurality of close-pitched threads 100 formed in the shaping portion 78. The forming tool 74 is pressed into the heated portion 70 of the polymeric film 68 when at malleable temperature, thereby forming the protrusion 80. Accordingly, the protrusion's sidewalls 86 are generally adjacent to the threads 100 on the forming tool 74, but do not yet closely conform to the threads. The differential pressure is then generated in the protrusion's interior 88, moving the sidewalls 86 to the contracted position and into close engagement with the threads 100, thereby forming the close-pitched, accurately positioned threads 102 in the protrusion. The forming tool 74 is then removed preferably by rotating it so the shaping portion 78 unscrews from the threads 102 formed in the protrusion 80.

The present invention also allows for easy and inexpensive formation of a thin-walled polymeric medical component 50, as shown in Figure 8, having precisely located detents 120 formed in selected positions that correspond to detent receiving portions on a selected medical device, not shown. The medical component 50 may also have a concave-shaped distal end 122 that follows the contour of the selected medical device. The medical component 50 illustrated in Figure 8 is formed using a forming tool 74 with a shaping portion 78 having one or more detent molds 124 in a selected position and having a concave distal end surface 125. The shaping portion 78 also has the vacuum ports 89 formed therein for creating the partial vacuum in the protrusion's interior portion 88. Accordingly, when the forming tool 74 is pressed into the film's heated portion 70 to form the protrusion 80 and the partial vacuum is drawn through the vacuum ports 89, the protrusion's sidewalls 86 are drawn into firm engagement with the forming tool's detent molds 124 so as to form the detents 120 in the protrusion. Similarly, the protrusion's closed distal end 84 is drawn into firm engagement with the forming tool's concave distal end 125, thereby forming a medical component 50 with a concave closed distal end.

After the polymeric material is cooled, the forming tool 74 is removed from the protrusion's interior 88 and the selected shaping portion of the protrusion 80 is cut in order to remove the complex-shaped medical component 50 from the rest of the protrusion. In the embodiment wherein a detent or the like is formed in the medical component 50, the forming tool 74 may be removed from the protrusion's interior 88 by slightly deflecting the sidewalls 86 of the protrusion 80 until sufficient clearance is provided between the sidewalls and the forming tool's shaping portion 78. Such deflection is possible because the thin-walled polymeric material is sufficiently flexible to move so as to allow removal of the forming tool without permanent deformation of the medical component 50.

In alternate embodiments, the thin-walled polymeric medical component 50 is formed by stretching or drawing the protrusion 80 in two or more steps, so selected portions of the component have a different thickness than the thickness of other portions of the component. In one embodiment, an endcap with an integral optical lens is formed by pressing a first forming tool 74 into the heated portion 70 to form the protrusion 80, and the differential pressure is applied to the protrusion, as discussed above. The first forming tool 74 is specifically designed to form the lens of the endcap to provide the selected thickness and optical characteristics of the lens.

After the lens is formed, the first forming tool is removed from the protrusion 80. While the protrusion 80 is still malleable, a second forming tool is pressed into the protrusion to further draw or stretch the protrusion's sidewalls 86 to form thinner sidewalls. The second forming tool is designed to support the lens portions so there is substantially no further drawing of the lens while the sidewalls 86 are being drawn. The differential pressure is exerted on the protrusion so the protrusion conforms to the second forming tool, and the second forming tool is then removed. Accordingly the lens maintains its selected thickness, and the sidewalls are thinner than when formed by the first forming tool.

In another one of the alternate embodiments, the protrusion 80 is formed with portions having different thicknesses by heating the film 68 a first time and forming the protrusion with a forming tool as discussed above. The protrusion 80 is then allowed to cool to below the malleable temperature. A selected portion of the protrusion 80, such as the sidewalls 86, is heated a second time to the malleable temperature, and the sidewalls or other selected portion is further stretched or drawn with the same or a different forming tool. Accordingly, the protrusion's selected portion that is reheated and further drawn is thinner while the protrusion's other portions that were not heated a second time are not further thinned by the second drawing process. If further drawing is needed at other specific portions of the protrusion 80, additional steps of heating and drawing can be performed to achieve the specific size, thickness, and characteristics of the component 50.In another alternate embodiment (not shown), the thin-walled polymeric medical component 50 is formed by pressing the forming tool 74 into the polymeric film's heated portion 70, such that the distal end of the forming tool pierces the distal end of the protrusion. In this alternate embodiment, the protrusion's sidewalls are substantially sealably connected to the forming tool's shaping portion 78, so a partial vacuum can still be generated to draw the sidewalls into firm engagement with the shaping portion.

In yet another embodiment (not shown), the complex-shaped medical component 50 is further modified or machined after formation of the component so as to remove or modify a selected portion of the medical component. As an example, polymeric material is removed from a distal end portion of a selected medical component to allow a selected medical device to be positioned partially within the component, while a selected portion of the device extends through the thin-walled polymeric medical component. Accordingly, the thin-walled, polymeric medical component can be formed with a selected complex shape for use with a wide range of medical devices.

From the foregoing it will be appreciated that, although specific embodiments of the invention have been described herein for purposes of illustration, various modifications may be made without deviating from the scope of the invention. Also, although the various embodiments of the invention have been described as being used to form complex components, it will be understood that relatively simple components may also be formed in accordance with the invention. Accordingly, the invention is not limited except as by the appended claims.

## Claims

1. A method of forming a polymeric component (50) having a generally C-shaped cross sectional shape, comprising the steps of:
retaining a sheet of polymeric film in a selected position;
heating the sheet of polymeric film (68) to a malleable temperature to form a heated portion (70) of the sheet;
pressing a shaping portion (78) of a forming tool (74) into the heated portion (70) while the heated portion (70) is malleable to form a protrusion (80) having an open proximal end (82), a distal end (84), and sidewalls (86) extending between the proximal and distal ends (82, 84), the sidewalls (86) and distal end (84) defining a first shape having a shape that is different from the shape of the shaping portion (78) of the forming tool (74), the shaping portion (78) of the forming tool (74) including a segment having a generally C-shaped cross-sectional shape; and exerting a differential pressure on the protrusion (80) to move the distal end (84) and sidewalls (86) inwardly to engage with the forming tool's shaping portion (78) with the distal end (84) and sidewalls (86) moving from the first shape to a second shape that conforms to the shape of the forming tool's shaping portion (78) and wherein at least part of the protrusion (80) has a cross-sectional shape that conforms with the generally C-shaped cross-sectional shape; and
cooling the protrusion (80) to a temperature below the malleable temperate and after the cooling step heating a selected portion of the protrusion (80) to the malleable temperature and changing the thickness of the selected portion of the protrusion (80) from a first thickness to a second thickness that is less than the first thickness.

2. A method of forming a polymeric component (50) having a complex shape, comprising the steps of:
retaining a sheet of polymeric film in a selected position;
heating the sheet of polymeric film (68) to a malleable temperature to form a heated portion (70) of the sheet;
pressing a shaping portion (78) of a forming tool (74) into the heated portion (70) while the heated portion (70) is malleable to form a protrusion (80) having an open proximal end (82), a distal end (84), and sidewalls (86) extending between the proximal and distal ends (82, 84), the sidewalls (86) and distal end (84) defining a first shape having a shape that is different from the shape of the shaping portion (78) of the forming tool (74), the shaping portion (78) of the forming tool (74) including a segment having a complex shape; and
exerting a differential pressure on the protrusion (80) to move the distal end (84) and sidewalls (86) inwardly to engage with the forming tool's shaping portion (78) with the distal end (84) and sidewalls (86) moving from the first shape to a second shape that conforms to the shape of the forming tool's shaping portion (78) and wherein at least part of the protrusion (80) has a cross-sectional shape that conforms with the complex shape of the shaping portion; and
removing the forming tool (74) from the protrusion (80) after the distal end (84) and sidewalls (86) have moved to the second shape, and inserting a second forming tool (74) into the protrusion (80) and changing the thickness of a selected portion of the protrusion (80) from a first thickness to a second thickness that is less that the first thickness.

3. The method of claim 1 or 2, further including the steps of cooling the protrusion (80) to a temperature below the malleable temperature, and removing the forming tool (74) from the protrusion (80) after the protrusion (80) has cooled.

4. The method of claim 1 or 2 wherein the step of pressing the shaping portion (78) of the forming tool (74) into the heated portion (70) includes moving the forming tool (74) against the heated portion (70) while malleable to form a generally funnel-shaped protrusion (80).

5. The method of claim 1 or 2 wherein the step of exerting a differential pressure on the protrusion (80) includes creating a partial vacuum within the protrusion (80) when the heated portion (70) is malleable to move the distal end (84) and sidewalls (86) into engagement with the forming tool's shaping portion (78).

6. The method of claim 1 or 2 wherein the forming tool (74) has at least one vacuum port (89) therein and is adapted to draw a partial vacuum therethrough when positioned within the protrusion (80), and the step of creating a partial vacuum within the protrusion (80) includes drawing the partial vacuum through the vacuum port (89).

7. The method of claim 1 or 2 wherein the step of exerting a differential pressure on the protrusion (80) includes creating an exterior pressure on the protrusion (80) when the heated portion (70) is malleable to press the distal end (84) and sidewalls (86) inwardly into the second shape.

8. The method of claim 1 or 2 wherein the forming tool's shaping portion (78) includes a detent mold (124) thereon, and the step of exerting a differential pressure on the protrusion (80) includes moving the sidewalls (86) into engagement with the detent molds (124) to form a detent member therein.

9. The method of claim 1 or 2 wherein the cross-sectional shape of the shaping portion of the forming tool is a generally C-shaped cross-section defined by a first outer axial surface (60) with a first radius and a second outer axial surface (62) with a second radius that is smaller than the first radius.

10. The method of claim 1 or 2 wherein the forming tool's shaping portion (78) has a plurality of first threads (100) thereon and the step of exerting a differential pressure on the protrusion (80) includes moving the protrusion's sidewalls (86) into engagement with the first threads (100) to form a plurality of second threads (102) in the sidewalls (86) that conform to the first threads (100) on the forming tool's shaping portion (78).

11. The method of claim 1 or 2 wherein the process further includes the step of forming an aperture in the distal end (84) of the protrusion (80).

12. The method of claims 1 or 2 wherein the distal end (84) of the protrusion (80) is a closed distal end (84) defining an optically transparent viewing window connectable to an endoscopic sheath.

13. An arrangement comprising an endcap (51), a medical device (56) and a sheath (54) that covers a portion of the medical device (56) having a selected cross-sectional shape, the endcap (51) comprising an open proximal end (82), a closed distal end (84), and sidewalls (86) defining an interior area (88) for receiving the portion of the medical device, the sidewalls (86) being connectable to the sheath (54), the sidewalls (86) having a thickness in the range of approximately 0.0127 mm (0.0005 inches) to 0.381 mm (0.015 inches), inclusive, the endcap's interior area having a cross-sectional shape that substantially corresponds to the medical device's (56) selected cross-sectional shape, wherein the closed distal end (84) is an optical lens (5) having a first thickness and the sidewall (86) has a second thickness that is less than the first thickness.

14. The endcap of claim 13 having a generally C-shaped cross-section defined by a first outer axial surface (60) with a first radius and a second outer axial surface (62) with a second radius that is smaller than the first radius.

## Patentansprüche

1. Verfahren des Ausbildens einer Polymerkomponente (50) mit einem im Wesentlichen C-förmigen Querschnitt, das die Schritte aufweist:
Halten eines Stücks einer Polymerfolie in einer ausgewählten Position;
Erwärmen des Stücks der Polymerfolie (68) auf eine Verformbarkeitstemperatur, um einen erwärmten Bereich (70) des Stücks auszubilden;
Drücken eines formgebenden Bereichs (78) eines Formungswerkstücks (74) in den erwärmten Bereich, während der erwärmte Bereich (17) verformbar ist, um einen hervorstehenden Bereich (80) auszubilden, der ein offenes proximales Ende (82), ein distales Ende (84) und Seitenwände (86) hat, die sich zwischen dem proximalen und dem distalen Ende (82, 84) erstrecken, wobei die Seitenwände (86) und das distale Ende (84) eine erste Form definieren, die eine andere Form als die Form des formgebenden Bereichs (78) des Formungswerkzeugs (74) hat, wobei der formgebende Bereich (78) des Formungswerkzeugs (74) ein Segment mit einer im Wesentlichen C-förmigen Querschnittsform aufweist; und
Ausüben eines Differenzialdrucks auf den hervorstehenden Bereich (80), um das distale Ende (84) und die Seitenwände (86) nach innen zu bewegen, damit sie in den formgebenden Bereich (78) des Formungswerkzeugs eingreifen, wobei sich das distale Ende (84) und die Seitenwände (86) von einer ersten Form in eine zweite Form bewegen, die mit der Form des formgebenden Bereichs (78) des Formungswerkzeugs übereinstimmt, und wobei wenigstens ein Bereich des hervorstehenden Bereichs (80) eine Querschnittsform hat, die mit der im Wesentlichen C-förmigen Querschnittsform übereinstimmt; und
Abkühlen des hervorstehenden Bereichs (80) auf eine Temperatur unterhalb der Verformbarkeitstemperatur und nach dem Schritt des Abkühlens Erwärmen eines ausgewählten Bereiches des hervorstehenden Bereichs (80) auf die Verformbarkeitstemperatur und Ändern der Dicke des ausgewählten Bereichs des hervorstehenden Bereichs (80) von einer ersten Dicke auf eine zweite Dicke, die dünner als die erste Dicke ist.

2. Verfahren des Ausbildens einer Polymerkomponenten (50) mit einer komplexen Form, das die Schritte aufweist:
Halten eines Stücks einer Polymerfolie in einer ausgewählten Position;
Erwärmen des Stücks der Polymerfolie (68) auf einer Verformbarkeitstemperatur, um einen erwärmten Bereich (70) des Stücks auszubilden;
Drücken eines formgebenden Bereichs (78) eines Formungswerkzeugs (74) in den erwärmten Bereich (70), während der erwärmte Bereich (70) verformbar ist, um einen hervorstehenden Bereich (80) auszubilden, der ein offenes proximales Ende (82), ein distales Ende (84) und Seitenwände (86) hat, die sich zwischen dem proximalen und dem distalen Ende (82, 84) erstrecken, wobei die Seitenwände (86) und das distale Ende (84) eine erste Form definieren, die eine andere Form als der formgebende Bereich (78) des Formungswerkzeugs (74) hat, wobei der formgebende Bereich (78) des Formungswerkzeugs (74) ein Segment mit einer komplexen Form aufweist; und
Ausüben eines Differenzialdrucks auf den hervorstehenden Bereich (80), um das distale Ende (84) und die Seitenwände (86) nach innen zu bewegen, damit sie in den formgebenden Bereich (78) des Formungswerkzeugs eingreifen, wobei sich das distale Ende (84) und die Seitenwände (86) von der ersten Form in eine zweite Form bewegen, die mit der Form des formgebenden Bereichs (78) des Formungswerkzeugs übereinstimmt, und wobei wenigstens ein Bereich des hervorstehenden Bereichs (80) eine Querschnittsform hat, die mit der komplexen Form des formgebenden Bereichs übereinstimmt; und
Entfernen des Formungswerkzeugs (74) von dem hervorstehenden Bereich (80), nachdem das distale Ende (84) und die Seitenwände (86) in die zweite Form bewegt worden sind, und Einführen eines zweiten Formungswerkzeugs (74) in den hervorstehenden Bereich (80) und Ändern der Dicke eines ausgewählten Bereichs des hervorstehenden Bereichs (80) von einer ersten Dicke auf eine zweite Dicke, die kleiner als die erste Dicke ist.

3. Verfahren nach Anspruch 1 oder 2, das zusätzlich die Schritte des Abkühlens des hervorstehenden Bereichs (80) auf eine Temperatur unterhalb der Verformbarkeitstemperatur und, nachdem der hervorstehende Bereich (80) abgekühlt worden ist, des Entfernens des Formungswerkzeugs (74) aus dem hervorstehenden Bereich (80) einschließt.

4. Verfahren nach Anspruch 1 oder 2, wobei der Schritt des Drückens des formgebenden Bereichs (78) des Formungswerkzeugs (74) in den erwärmten Bereich (70) einschließt, das Formungswerkzeug (74) gegenüber dem erwärmten Bereich (70) zu bewegen, während dieser verformbar ist, um einen im Wesentlichen trichterförmigen hervorstehenden Bereich (80) auszubilden.

5. Verfahren nach Anspruch 1 oder 2, wobei das Ausüben eines Differenzialdrucks auf den hervorstehenden Bereich (80) einschließt, innerhalb des hervorstehenden Bereichs (80) ein Teilvakuum zu erzeugen, wenn der erwärmte Bereich (70) verformbar ist, um das distale Ende (84) und die Seitenwände (86) in Eingriff mit dem formgebenden Bereich (78) des Formungswerkzeugs zu bewegen.

6. Verfahren nach Anspruch 1 oder 2, wobei in dem Formungswerkzeug (74) wenigstens einen Vakuumanschluss (89) vorhanden und eingerichtet ist, um, während es in dem hervorstehenden Bereich (80) angeordnet ist, durch Saugen ein Teilvakuum zu erzeugen, und der Schritt des Erzeugens eines Teilvakuums innerhalb des hervorstehenden Bereichs (80) einschließt, das Teilvakuum durch Saugen durch den Vakuumanschluss (89) herzustellen.

7. Verfahren nach Anspruch 1 oder 2, wobei der Schritt des Ausübens eines Differenzialdrucks auf den hervorstehenden Bereich (80) einschließt, einen äußeren Druck auf den hervorstehenden Bereich (80) zu erzeugen, wenn der erwärmte Bereich (70) verformbar ist, um das distale Ende (84) und die Seitenwände (86) in die zweite Form nach innen zu drücken.

8. Verfahren nach Anspruch 1 oder 2, wobei der formgebende Bereich (78) des Formungswerkzeugs eine Arretierungsform (124) aufweist, und der Schritt des Ausübens eines Differenzialdrucks auf den hervorstehenden Bereich (80) einschließt, die Seitenwände (86) in Eingriff mit den Arretierungsformen (124) zu bringen, um darin ein Arretierungselement auszubilden.

9. Verfahren nach Anspruch 1 oder 2, wobei der Querschnitt des formgebenden Bereichs des Formungswerkzeugs ein im Wesentlichen C-förmiger Querschnitt ist, der durch eine erste äußere axiale Fläche (60) mit einem ersten Radius und eine zweite äußere axiale Fläche (62) mit einem zweiten Radius, der kleiner als der erste Radius ist, definiert wird.

10. Verfahren nach Anspruch 1 oder 2, wobei auf dem formgebenden Bereich (78) des Formungswerkzeugs mehrere erste Rillen ausgebildet sind, und der Schritt des Ausübens eines Differenzialdrucks auf den hervorstehenden Bereich (80) einschließt, die Seitenwände (86) des hervorstehenden Bereichs in Eingriff mit den ersten Rillen (100) zu bringen, um in den Seitenwänden (86) mehrere zweite Rillen (102) auszubilden, die den ersten Rillen (100) auf dem formgebenden Bereich (78) des Formungswerkzeugs entsprechen.

11. Verfahren nach Anspruch 1 oder 2, wobei das Verfahren zusätzlich den Schritt einschließt, im distalen Ende (84) des hervorstehenden Bereichs (80) eine Öffnung auszubilden.

12. Verfahren nach Anspruch 1 oder 2, wobei das distale Ende (84) des hervorstehenden Bereichs (80) ein geschlossenes distales Ende (84) ist, das ein optisch transparentes Sichtfenster definiert, das mit einer endoskopischen Hülle verbindbar ist.

13. Anordnung mit einer Endkappe (51), einem Medizingerät (56) und einer Hülse (54), die einen Bereich des Medizingeräts (56), das eine ausgewählte Querschnittsform hat, bedeckt, wobei die Endkappe (51) ein offenes proximales Ende (82), ein geschlossenes distales Ende (84) und Seitenwände (86) aufweist, die einen Innenbereich (88) definieren, der ausgebildet ist, um den Bereich des Medizingeräts aufzunehmen, wobei die Seitenwände (86) mit der Hülse (54) verbindbar sind und eine Dicke im Bereich von ungefähr 0,0127 mm (0,0005 Zoll) bis 0,381 mm (0,015 Zoll) haben, wobei der Innenbereich der Endkappe einen Querschnitt hat, der im Wesentlichen dem ausgewählten Querschnitt des medizinischen Geräts (56) entspricht, und wobei das geschlossenen distale Ende (84) eine optische Linse mit einer ersten Dicke ist und die Seitenwand (86) eine zweite Dicke hat, die kleiner als die erste Dicke ist.

14. Endkappe nach Anspruch 13 mit einem im Wesentlichen C-förmigen Querschnitt, der durch eine erste äußere Axialfläche (60) mit einem ersten Radius und eine zweite äußere Axialfläche (62) mit einem zweiten Radius, der kleiner als der erste Radius ist, definiert wird.

## Revendications

1. Procédé de façonnement d'un composant polymère (50) possédant une configuration en coupe transversale généralement en forme de C, comprenant les étapes consistant à :
maintenir une feuille d'un film polymère dans une position sélectionnée ;
chauffer la feuille de film polymère (68) jusqu'à une température de malléabilité pour obtenir une portion chauffée (70) de la feuille ;
introduire par compression une portion de configuration (78) d'un outil de façonnement (74) dans la portion chauffée (70) lorsque la portion chauffée. (70) est malléable pour obtenir une protubérance (80) possédant une extrémité proximale ouverte (82), une extrémité distale (84) et des parois latérales (86) qui s'étendent entre les extrémités proximale et distale (82, 84), les parois latérales (86) et l'extrémité distale (84) définissant une première configuration dont la forme est différente de la forme de la portion de configuration (78) de l'outil de façonnement (74), la portion de façonnement (78) de l'outil de façonnement (74) englobant un segment possédant une configuration en coupe transversale généralement en forme de C ; et
exercer une pression différentielle sur la protubérance (80) afin de déplacer l'extrémité distale (84) et les parois latérales (86) vers l'intérieur pour une mise en contact avec la portion de configuration (78) de l'outil de façonnement, l'extrémité distale (84) et les parois latérales (86) passant de la première configuration à une deuxième configuration qui épouse la configuration de la portion de configuration (78) de l'outil de façonnement et au moins une partie de la protubérance (80) possédant une configuration en coupe transversale qui épouse la configuration en coupe transversale généralement en forme de C ; et
refroidir la protubérance (80) jusqu'à une température inférieure à la température de malléabilité et, après l'étape de refroidissement, chauffer une portion sélectionnée de la protubérance (80) jusqu'à la température de malléabilité, et modifier l'épaisseur de la portion sélectionnée de la protubérance (80) en passant d'une première épaisseur à une deuxième épaisseur qui est inférieure à la première épaisseur.

2. Procédé de formation d'un composant polymère (50) possédant une configuration complexe, comprenant les étapes consistant à :
maintenir une feuille d'un film polymère dans une position sélectionnée ;
chauffer la feuille de film polymère (68) jusqu'à une température de malléabilité pour obtenir une portion chauffée (70) de la feuille ;
introduire par compression une portion de configuration (78) d'un outil de façonnement (74) dans la portion chauffée (70) lorsque la portion chauffée (70) est malléable pour obtenir une protubérance (80) possédant une extrémité proximale ouverte (82), une extrémité distale (84) et des parois latérales (86) qui s'étendent entre les extrémités proximale et distale (82, 84), les parois latérales (86) et l'extrémité distale (84) définissant une première configuration dont la forme est différente de la forme de la portion de configuration (78) de l'outil de façonnement (74), la portion de façonnement (78) de l'outil de façonnement (74) englobant un segment possédant une configuration complexe ; et
exercer une pression différentielle sur la protubérance (80) afin de déplacer l'extrémité distale (84) et les parois latérales (86) vers l'intérieur pour une mise en contact avec la portion de configuration (78) de l'outil de façonnement, l'extrémité distale (84) et les parois latérales (86) passant de la première configuration à une deuxième configuration qui épouse la configuration de la portion de configuration (78) de l'outil de façonnement et au moins une partie de la protubérance (80) possédant une configuration en coupe transversale qui épouse la configuration complexe de la portion de configuration ; et
retirer l'outil de façonnement (74) de la protubérance (80) après le passage de l'extrémité distale (84) et des parois latérales (86) à la seconde configuration, et insérer un deuxième outil de façonnement (74) dans la protubérance (80) et modifier l'épaisseur de la portion sélectionnée de la protubérance (80) en passant d'une première épaisseur à une deuxième épaisseur qui est inférieure à la première épaisseur.

3. Procédé selon la revendication 1 ou 2, englobant en outre les étapes consistant à refroidir la protubérance (80) jusqu'à une température inférieure à la température de malléabilité, et retirer l'outil de façonnement (74) de la protubérance (80) après le refroidissement de la protubérance (80).

4. Procédé selon la revendication 1 ou 2, dans lequel l'étape consistant à introduire par compression la portion de configuration (78) de l'outil de façonnement (74) dans la portion chauffée (70) englobe le fait de déplacer l'outil de façonnement (74) jusque contre la portion chauffée (70) lorsque celle-ci est malléable pour obtenir une protubérance (80) généralement en forme d'entonnoir.

5. Procédé selon la revendication 1 ou 2, dans lequel l'étape consistant à exercer une pression différentielle sur la protubérance (80), englobe le fait de créer un vide partiel au sein de la protubérance (80) lorsque la portion chauffée (70) est malléable afin d'amener l'extrémité distale (84) et les parois latérales (86) en contact avec la portion de configuration (78) de l'outil de façonnement.

6. Procédé selon la revendication 1 ou 2, dans lequel l'outil de façonnement (74) possède au moins un orifice pour le vide (89) et est conçu pour appliquer un vide partiel à travers l'orifice en question lorsqu'il est disposé au sein de la protubérance (80), et l'étape consistant à créer un vide partiel au sein de la protubérance (80) englobe le fait d'appliquer le vide partiel à travers l'orifice pour le vide (89).

7. Procédé selon la revendication 1 ou 2, dans lequel l'étape consistant à exercer une pression différentielle sur la protubérance (80), englobe le fait d'exercer une pression externe sur la protubérance (80) lorsque la portion chauffée (70) est malléable pour permettre à l'extrémité distale (84) et aux parois latérales (86) de prendre leur deuxième configuration par compression vers l'intérieur.

8. Procédé selon la revendication 1 ou 2, dans lequel un moule d'encliquetage (124) est disposé sur la portion de configuration (78) de l'outil de façonnement, et l'étape consistant à exercer une pression différentielle sur la protubérance (80) englobe la mise des parois latérales (86) en contact avec le moule d'encliquetage (124) pour y former un membre d'encliquetage.

9. Procédé selon la revendication 1 ou 2, dans lequel la configuration en section transversale de la portion de configuration de l'outil de façonnement est une section transversale généralement en forme de C définie par une première surface axiale externe (60) possédant un premier rayon et par une deuxième surface axiale externe (62) possédant un deuxième rayon qui est inférieur au premier rayon.

10. Procédé selon la revendication 1 ou 2, dans lequel la portion de configuration (78) de l'outil de façonnement possède plusieurs premiers filetages (100) et l'étape consistant à exercer une pression différentielle sur la protubérance (80) englobe la mise des parois latérales (86) de la protubérance en engrènement avec les premiers filetages (100) pour obtenir plusieurs deuxièmes filetages (102) dans les parois latérales (86) qui épousent la configuration des premiers filetages (100) sur la portion de configuration (78) de l'outil de façonnement.

11. Procédé selon la revendication 1 ou 2, dans lequel le procédé englobe en outre l'étape consistant à former un orifice dans l'extrémité distale (84) de la protubérance (80).

12. Procédé selon la revendication 1 ou 2, dans lequel l'extrémité distale (84) de la protubérance (80) est une extrémité distale fermée (84) définissant une fenêtre d'observation transparente du point de vue optique qui peut venir se raccorder à une gaine endoscopique.

13. Arrangement comprenant un capuchon terminal (51), un dispositif médical (56) et une gaine (54) qui recouvre une portion du dispositif médical (56) possédant une configuration sélectionnée en section transversale, le capuchon terminal (51) comprenant une extrémité proximale ouverte (82), une extrémité distale fermée (84) et des parois latérales (86) définissant une zone interne (88) pour la réception de la portion du dispositif médical, les parois latérales (86) pouvant se raccorder à la gaine (54), les parois latérales (86) possédant une épaisseur dans la plage d'approximativement 0,0127 mm (0,0005 pouce) à 0,381 mm (0,05 pouce) inclus, la zone interne du capuchon terminal possédant une configuration en section transversale qui correspond essentiellement à la configuration sélectionnée du dispositif médical (56) en section transversale, l'extrémité distale fermée (84) représentant une lentille optique (5) possédant une première épaisseur et la paroi latérale (86) possédant une deuxième épaisseur qui est inférieure à la première épaisseur.

14. Capuchon terminal selon la revendication 13, possédant une section transversale généralement en forme de C définie par une première surface axiale externe (60) possédant un premier rayon et par une deuxième surface axiale externe (62) possédant un deuxième rayon qui est inférieur au premier rayon.
